(19) Europäisches Patentamt / European Patent Office / Office européen des brevets

(11) **EP 4 026 577 B1**

(12) **FASCICULE DE BREVET EUROPEEN**

(45) Date de publication et mention de la délivrance du brevet:
**12.07.2023 Bulletin 2023/28**

(21) Numéro de dépôt: **21209030.2**

(22) Date de dépôt: **18.11.2021**

(51) Classification Internationale des Brevets (IPC):
**A61M 16/00** (2006.01)

(52) Classification Coopérative des Brevets (CPC):
**A61M 16/024;** A61M 16/0051; A61M 16/0078;
A61M 16/06; A61M 16/0858; A61M 16/1005;
A61M 16/12; A61M 16/204; A61M 16/208;
A61M 2016/0018; A61M 2016/0027;
A61M 2016/0039; A61M 2202/02; A61M 2202/0208;
A61M 2202/0225; (Cont.)

(54) **INSTALLATION DE FOURNITURE DE GAZ THÉRAPEUTIQUE À UN PATIENT AVEC PRISE EN COMPTE DES DÉFAUTS D'ÉTANCHÉITÉ AU MASQUE**

ANLAGE ZUR VERSORGUNG EINES PATIENTEN MIT THERAPEUTISCHEM GAS UNTER BERÜCKSICHTIGUNG VON UNDICHTIGKEITEN AN DER MASKE

INSTALLATION FOR THE SUPPLY OF THERAPEUTIC GAS TO A PATIENT WITH CONSIDERATION OF MASK LEAKAGE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.01.2021 FR 2100102**

(43) Date de publication de la demande:
**13.07.2022 Bulletin 2022/28**

(73) Titulaire: **L'Air Liquide, société anonyme pour l'Étude
et l'Exploitation des procédés Georges Claude
75007 Paris (FR)**

(72) Inventeur: **BOULANGER, Thierry
Newark, 19702 (US)**

(74) Mandataire: **Air Liquide
L'Air Liquide S.A.
Direction de la Propriété Intellectuelle
75, Quai d'Orsay
75321 Paris Cedex 07 (FR)**

(56) Documents cités:
**EP-A1- 3 701 992 WO-A1-99/61090
CN-A- 105 771 049**

(52) Classification Coopérative des Brevets (CPC):
(Cont.)
A61M 2205/0216; A61M 2205/3334;
A61M 2205/505; A61M 2205/8206

**Description**

L'invention concerne une installation de fourniture de gaz thérapeutique comprenant un appareil de délivrance de gaz et un masque respiratoire à port de sortie de gaz expiré servant à fournir un gaz thérapeutique (i.e. gaz pur ou mélange gazeux) à un patient prenant en compte les éventuelles fuites au niveau de l'interface respiratoire, tel qu'un masque facial, c'est-à-dire tout défaut d'étanchéité fluidique sur le pourtour de l'interface respiratoire, et une éventuelle dilution du gaz thérapeutique par de l'air ambiant entrant dans le masque du fait desdites fuites.

Certaines thérapies nécessitent l'administration à des patients conscients de gaz thérapeutique formé d'un mélange de plusieurs constituants gazeux. Ainsi, il est connu d'utiliser un mélange équimolaire (50%/50%) de protoxyde d'azote ($N_2O$) et d'oxygène ($O_2$) pour amoindrir les états d'anxiété, produire un effet sédatif et/ou atténuer la douleur aigue. De même, il a été proposé d'utiliser un mélange d'argon et d'oxygène (60 vol.% Ar/40 vol.% $O_2$), inhalé avant et pendant, voire après, une procédure de thrombectomie mécanique, afin de traiter les accidents vasculaires cérébraux ou AVC.

L'inhalation du gaz thérapeutique (i.e. 1 ou plusieurs constituants) à un patient se fait en général via un masque respiratoire, typiquement un masque facial, i.e. naso-buccal, de façon continue ou intermittente.

Pendant une administration continue, un débit continu de gaz, excédant la ventilation minute du patient (i.e. le volume moyen de gaz inhalé par le patient pendant une minute) est délivré en continu pendant les phases inspiratoires et expiratoires du patient. Pendant les phases inspiratoires, le patient inhale le gaz contenu dans un réservoir déformable, alors que pendant les phases expiratoires, le gaz rempli à nouveau le réservoir déformable pour préparer la phase inspiratoire suivante.

Toutefois, l'administration du gaz se heurte à un problème lors du traitement de certaines pathologies, par exemple lors d'une thrombectomie mécanique mise en place dans le traitement des AVC. En effet, du fait de contraintes d'espace relatives au traitement de ces pathologies, l'appareil de délivrance de gaz doit être placée généralement assez loin de la tête du patient au sein de la salle d'opération ou analogue, et raccordée fluidiquement au masque distribuant le gaz audit patient via une ou des tubulures d'amenée de gaz, c'est-à-dire un ou des tuyaux flexibles de plusieurs mètres de longueur, typiquement environ 3 ou 4 mètres, ou plus.

Or, du fait de leur longueur, ces tubulures d'amenée de gaz engendrent une résistance supplémentaire à l'inspiration, c'est-à-dire que le patient doit produire une pression négative (i.e. une dépression) d'autant plus importante pour pouvoir inspirer du gaz et subvenir à ses besoins ventilatoires que la résistance des tubulures est élevée.

En cas de « fuites » au niveau du masque, c'est-à-dire de défauts d'étanchéité fluidique sur le pourtour du masque en contact avec le visage du patient, cette pression négative entraine de l'air ambiant qui pénètre alors dans le masque et dilue le gaz inhalé, pendant les phases inspiratoires du patient.

Cette dilution non-désirée du gaz thérapeutique par de l'air ambiant est très problématique car elle peut conduire à une baisse d'efficacité notable du médicament gaz, i.e. du gaz thérapeutique, administré au patient. Par exemple, les propriétés thérapeutiques de l'argon s'estompent dès lors que sa concentration devient inférieure à 50 vol. %.

Ceci n'est pas acceptable au plan médical et il est alors nécessaire de s'assurer que la dilution du gaz thérapeutique n'est pas excessive, au risque de ne pas obtenir le bénéfice clinique escompté, c'est-à-dire de ne pas traiter efficacement le patient.

Cependant, comme les défauts d'étanchéité sur le pourtour du masque en contact avec le visage du patient ne sont pas constants car extrêmement variables d'un patient à l'autre, en particulier du fait de différences de morphologie anatomique, de la présence ou non d'une barbe, ou autres, il n'est pas possible de savoir d'avance si la dilution du gaz thérapeutique par l'air ambient engendrera une dilution du gaz thérapeutique faible ou, au contraire excessive, chez un patient donné.

EP-A-3701992 propose un appareil de délivrance de gaz comprenant un passage de gaz, un réservoir flexible et une unité à processeur. Un capteur de pression différentiel sert à mesurer la pression dans le réservoir. Une vanne proportionnelle permet d'ajuster le débit dans le passage de gaz en fonction des mesures de pression opérées par le capteur de pression différentiel. La question des fuites au masque et de la dilution du gaz thérapeutique qui en résulte n'y est pas traitée.

Dans ce contexte, le problème est de proposer une installation de fourniture de gaz thérapeutique, i.e. gaz pur ou mélange gazeux, à un patient comprenant une source de gaz, un appareil de délivrance de gaz alimentant un masque respiratoire avec du gaz thérapeutique provenant de la source de gaz, dans laquelle les fuites au masque sont évaluées et prises en compte afin de pouvoir garantir que le gaz thérapeutique inhalé par le patient est à une concentration suffisante, c'est-à-dire efficace, même en cas de dilution non-désirée par de l'air ambiant entrant dans le masque via des défauts d'étanchéité, i.e. des fuites au masque, et, le cas échéant, d'avertir l'utilisateur en cas de dilution excessive conduisant à une concentration non-efficace du gaz thérapeutique.

Une solution selon l'invention concerne alors une installation de fourniture de gaz thérapeutique à un patient comprenant :

- une source de gaz comprenant au moins un composé thérapeutiquement actif à une concentration initiale ($C_{ini}$) donnée,
- un appareil de délivrance de gaz alimenté en gaz par ladite source de gaz, et
- une interface respiratoire munie d'au moins un port expiratoire alimenté en gaz par ledit appareil de délivrance de gaz,

et dans laquelle l'appareil de délivrance de gaz comprend :

- un passage de gaz interne en communication fluidique avec un réservoir déformable pour alimenter le réservoir déformable en gaz,
- un dispositif à vanne agencée sur le passage de gaz interne, en amont du réservoir déformable, pour contrôler le débit de gaz circulant dans le passage de gaz interne,
- une unité de contrôle à microprocesseur pilotant le dispositif à vanne pour contrôler le débit de gaz traversant le dispositif à vanne et alimentant le réservoir déformable en gaz,
- un capteur de pression configuré pour opérer une ou des mesures de pression de gaz ($P_{masque}$) au niveau de l'interface respiratoire et fournir à l'unité de contrôle, la ou lesdites mesures de pression de gaz ($P_{masque}$),
- un capteur de débit agencé dans le passage de gaz interne pour mesurer le débit de gaz fourni ($Q_{alim}$) circulant dans ledit passage de gaz interne et fournir la ou lesdites mesures de débit de gaz (Q) à l'unité de contrôle, et
- des moyens d'alarme, en particulier d'alarme sonore et/ou visuelle.

De plus, l'appareil de délivrance de gaz de l'installation de fourniture de gaz thérapeutique de l'invention comprend en outre des moyens de mémorisation pour mémoriser une valeur-seuil de concentration ($C_{min}$) en composé thérapeutiquement actif et l'unité de contrôle est configurée pour :

- déterminer le débit expiré ($Q_{exp}$) s'échappant par le port expiratoire de l'interface respiratoire à partir de la pression de gaz ($P_{masque}$) mesurée au niveau de l'interface respiratoire, pendant au moins une phase expiratoire dudit patient,
- déterminer à partir du débit de gaz fourni ($Q_{alim}$) pendant une phase inspiratoire du patient et du débit expiré ($Q_{exp}$) pendant la phase expiratoire suivant ladite phase inspiratoire du patient, des volumes de gaz fourni ($V_{alim}$) et expiré ($V_{exp}$),
- déterminer à partir des volumes de gaz fourni ($V_{alim}$) et expiré ($V_{exp}$), de l'intégrale de la pression de gaz ($P_{masque}$) pendant la phase inspiratoire et de l'intégrale de la pression de gaz ($P_{masque}$) pendant la phase expiratoire, au moins un volume de fuite ($V_{fuite}$) entrant dans l'interface respiratoire pendant ladite phase inspiratoire,
- déterminer à partir du volume de fuite ($V_{fuite}$) et des volumes de gaz fourni ($V_{alim}$) et expiré ($V_{exp}$), un taux de dilution du gaz,
- calculer la concentration réelle ($C_{reel}$) en ledit au moins un composé thérapeutiquement actif dans de l'interface respiratoire à partir de la concentration initiale ($C_{ini}$) dudit au moins un composé thérapeutiquement actif et du taux de dilution du gaz déterminé,
- comparer la concentration réelle calculée ($C_{reel}$) à la valeur-seuil de concentration ($C_{min}$) en composé thérapeutiquement actif mémorisée par les moyens de mémorisation, et
- commander les moyens d'alarme pour déclencher une alarme sonore et/ou visuelle lorsque la concentration réelle ($C_{reel}$) calculée est inférieure à ladite valeur-seuil de concentration ($C_{min}$) en composé thérapeutiquement actif mémorisée.

Dans le cadre de l'invention :

- le terme « pression » est utilisé pour désigner de façon générale, une pression positive (> 0 bar), nulle (= 0 bar) ou négative (< 0 bar), c'est-à-dire une dépression.
- les pressions sont exprimées en bar ou mbar relatifs.
- le signe « - » devant une valeur de pression signifie que la pression est négative, c'est-à-dire qu'il s'agit d'une dépression (i.e. inférieure à la pression atmosphérique).
- le signe « + » devant une valeur de pression signifie que la pression est positive (i.e. supérieure à la pression atmosphérique).
- le terme « gaz thérapeutique » désigne un gaz à un ou plusieurs constituants ou composés gazeux, c'est-à-dire un gaz 'pur' ou un mélange gazeux, dont au moins un composé ou constituant est thérapeutiquement actif et/ou efficace.
- dans « unité de commande », le terme « unité » est équivalent aux termes « dispositifs », « appareils », « moyens », « système » ou analogues, et
- le terme « contrôle » est équivalent aux termes « commande », « pilotage », « traitement » ou similaire.

Selon le mode de réalisation considéré, l'installation de l'invention peut comprendre l'une ou plusieurs des caractéristiques suivantes :

- la source de gaz comprend de l'argon en tant que composé thérapeutiquement actif à une concentration initiale ($C_{ini}$) supérieure à 50% en volume, de préférence un mélange argon/oxygène.
- le mélange argon/oxygène contient de 35 à 45% vol.% $O_2$ et de 55 à 65% vol.% Ar, de préférence un mélange contenant de 38 à 43% vol.% $O_2$ et de 57 à 62% vol.% Ar, en particulier un mélange formé de 40% vol.% $O_2$ et 60% vol.% Ar.
- la concentration initiale ($C_{ini}$) est mémorisée, par exemple par un microprocesseur de unité de contrôle ou par une mémoire de stockage.
- la concentration initiale ($C_{ini}$) peut être fixée, c'est-à-dire indiquée ou entrée dans l'appareil, via une interface homme-machine ou HIM.
- la valeur-seuil de concentration ($C_{min}$) mémorisée est égale à 50% en volume.
- la valeur-seuil de concentration ($C_{min}$) mémorisée l'est par les moyens de mémorisation.
- les moyens de mémorisation comprennent une mémoire flash.
- l'unité de contrôle de l'appareil de délivrance de gaz est configurée pour déterminer le débit expiré ($Q_{exp}$) à partir de la pression de gaz ($P_{masque}$) mesurée au niveau de l'interface respiratoire et d'au moins une table de conversion mémorisée.
- lorsque l'unité de contrôle détermine que la pression de gaz ($P_{masque}$) mesurée dans l'interface respiratoire est inférieure ou égale à une valeur-seuil de pression donnée ($P_{seuil}$), i.e. $P_{masque} \leq P_{seuil}$, ladite unité de contrôle est configurée pour commander le dispositif à vanne pour augmenter le débit de gaz thérapeutique traversant ledit dispositif à vanne et alimentant le réservoir déformable.
- la valeur-seuil de pression ($P_{seuil}$) est inférieure ou égale à 0 mbar.
- la valeur-seuil de pression ($P_{seuil}$) est inférieure ou égale à -0.25 mbar, de préférence inférieure ou égale à -0.5 mbar.
- la valeur-seuil de pression ($P_{seuil}$) est mémorisée au sein de l'unité de contrôle.
- la valeur-seuil de pression ($P_{seuil}$) est mémorisée par le microprocesseur ou par une mémoire de stockage de données.
- la valeur-seuil de pression ($P_{seuil}$) est réglable.
- le passage de gaz interne comprend un ou plusieurs conduits, tuyaux ou analogue.
- le capteur de pression est ou comprend un capteur de pression différentiel.
- le capteur de pression est relié électriquement à l'unité de contrôle.
- le dispositif à vanne comprend une vanne proportionnelle.
- le capteur de pression est configuré pour fournir à l'unité de contrôle une ou des mesures de pression de gaz ($P_{masque}$), préférentiellement plusieurs mesures de pression successives, sous forme de valeurs numériques ou de signaux représentatifs de telles valeurs numériques (par ex. des signaux de tensions lesquels valeurs ou signaux peuvent être traités tels quels ou convertis en valeurs numériques par l'unité de contrôle.
- le capteur de pression est relié pneumatiquement, via une liaison pneumatique, tel un conduit flexible, à un masque respiratoire pour réaliser des mesures de pression dans la chambre respiratoire interne du corps de l'interface respiratoire, e.g. un masque facial.
- il comprend une source d'alimentation électrique de type cordon et prise d'alimentation au secteur (e.g. 110/220 V) et/ou une batterie interne, de préférence rechargeable.
- la source d'alimentation électrique alimente en courant électrique l'unité de contrôle et tous les autres composants de l'appareil présents (en fonction du mode de réalisation choisi) qui nécessitent de la puissance électrique pour fonctionner, par exemple un ou des composants de type écran d'affichage, LED, dispositif d'alarme sonore et/ou visuelle...
- il comprend un boitier externe rigide, par exemple en matériau polymère ou autre.
- l'unité de contrôle, au moins une partie du passage de gaz interne, le réservoir déformable, le capteur de pression et/ou le dispositif à vanne sont agencés dans le boitier.
- le réservoir déformable comprend un ballon flexible ou analogue.
- le réservoir déformable se déforme en fonction de la quantité et/ou la pression de gaz thérapeutique qu'il contient. Il peut donc adopter différents états, stades ou niveaux de remplissage, notamment un stade dit « plein », un stade dit « vide » (i.e. quantité résiduelle minimale de gaz) et des stades intermédiaires correspondant à un remplissage partiel du réservoir (i.e. entre les stades « plein » et « vide »).
- le capteur de pression est configuré ou contrôlé pour opérer des mesures de pression ($P_{masque}$) à des intervalles de temps donnés, de préférence toutes les 20 msec ou moins, préférentiellement toutes les 10 msec ou moins, voire toutes les 5 msec ou moins.
- l'unité de contrôle est configurée pour piloter le dispositif à vanne, en particulier la vanne proportionnelle, pour ajuster (i.e. fixer ou modifier) le débit de gaz traversant ledit dispositif à vanne en fonction de la comparaison faite par l'unité de contrôle entre la pression ($P_{masque}$) mesurée à l'interface respiratoire, c'est-à-dire au masque, et la valeur-seuil de pression donnée ($P_{seuil}$) préfixée servant de pression référence, en particulier pour augmenter le débit de gaz thérapeutique traversant le dispositif à vanne et alimentant le réservoir déformable, lorsque l'unité de contrôle détermine que la pression de gaz ($P_{masque}$) mesurée dans le masque est inférieure ou égale à la valeur-seuil de pression donnée ($P_{seuil}$), i.e. $P_{masque} \leq P_{seuil}$, avec $P_{seuil} \leq 0$ mbar, de préférence $P_{seuil} \leq$ -0.25 mbar, de manière à accélérer le remplissage dudit réservoir déformable.

- le réservoir déformable est en matériau flexible de type caoutchouc, silicone ou analogue, par exemple un caoutchouc-silicone LSR NuSil.
- l'unité de contrôle à microprocesseur comprend un ou plusieurs microprocesseurs, de préférence un (ou des) microcontrôleur.
- le (ou les) microprocesseur(s) met en oeuvre un ou plusieurs algorithmes.
- l'unité de contrôle comprend une ou plusieurs mémoires de stockage de données ou autres, par exemple des tables de référence.
- l'unité de contrôle à microprocesseur comprend une carte électronique portant le ou les microprocesseurs, de préférence un ou des microcontrôleurs.
- le débitmètre ou capteur de débit est agencé dans le passage de gaz interne pour mesurer le débit de gaz circulant dans ledit passage de gaz interne.
- le capteur de débit (i.e. débitmètre) agencé dans le passage de gaz interne, en aval du dispositif à vanne, en particulier la vanne proportionnelle, de manière à pouvoir mesurer le débit de gaz fourni par ledit dispositif à vanne.
- le capteur de débit est agencé en amont du réservoir déformable, de préférence en amont du point de raccordement de la ligne d'entrée d'air.
- le capteur de débit est relié électriquement à l'unité de contrôle et lui fournit les mesures qu'il opère.
- le capteur de débit est ou comprend un capteur de débit massique ou un capteur de pression différentielle.
- il comprend un dispositif anti-retour agencé dans le passage de gaz interne, en aval du réservoir, de préférence un clapet anti-retour.
- le réservoir déformable a un volume compris entre environ 0.1 et 3 L, mesuré au repos (i.e. pression interne égale à la pression atmosphérique).
- le réservoir déformable a une paroi d'épaisseur comprise entre 0.10 et 0.90 mm, typiquement entre 0.25 et 0.75 mm.
- l'appareil comprend en outre une (ou des) valve unidirectionnelle agencée dans le passage de gaz interne, en particulier en aval du réservoir déformable.
- l'appareil comprend en outre une interface homme-machine (IHM) comprenant un écran de visualisation d'informations, de préférence un écran tactile, et/ou une ou des touches ou boutons de sélection, notamment des touches virtuelles s'affichant sur l'écran tactile, et/ou un dispositif de mise en route, tel un bouton allumé/éteint ou « on/off », et/ou d'autres éléments.
- les moyens ou système d'alarme sont en outre configurer pour alerter l'utilisateur en cas de problème affectant l'appareil ou le gaz, par exemple un défaut de vanne ou de capteur, une mauvaise composition gazeuse (e.g. mélange hypoxique) ou autres.
- les moyens ou système d'alarme peut comprendre des moyens ou un dispositif d'émission de signaux sonores et/ou visuels.
- l'interface respiratoire est un masque respiratoire.
- le masque respiratoire est un masque facial (i.e. naso-buccal) couvrant le nez et la bouche du patient, en utilisation, c'est-à-dire lorsqu'il est porté par ledit patient.
- l'interface respiratoire, typiquement un masque, comprend un coussinet flexible venant au contact du visage du patient venant se positionner sur le nez et/ou la bouche du patient.
- l'interface respiratoire est un masque respiratoire comprenant un coussinet flexible porté par un corps de masque rigide.
- l'interface respiratoire est un masque respiratoire comprenant une ou des sangles de portage permettant de le fixer sur la tête du patient, typiquement un harnais ou analogue.
- la source de gaz thérapeutique comprend un ou plusieurs récipients de gaz, notamment des bouteilles.
- la source de gaz thérapeutique comprend un récipient de gaz contenant un mélange gazeux $O_2/N_2O$, de préférence un mélange équimolaire de $O_2/N_2O$ (i.e. 50 mol.%/50 mol.%).
- alternativement, la source de gaz thérapeutique comprend un récipient de gaz contenant le mélange gazeux binaire $O_2$/argon.
- alternativement, la source de gaz thérapeutique comprend un premier récipient de gaz contenant de l'argon ou du $N_2O$, un second récipient de gaz contenant de l'oxygène ($O_2$) et un mélangeur de gaz alimenté en gaz par lesdits premier et second récipients de gaz, ledit mélangeur opérant un mélange des gaz provenant des premier et second récipients de gaz pour obtenir un mélange gazeux $O_2/N_2O$ ou $O_2$/argon.
- elle comprend un conduit d'amenée de pression, i.e. une liaison pneumatique, agencé entre le masque respiratoire et le capteur de pression de l'appareil de délivrance de gaz.

L'invention va maintenant être mieux comprise grâce à la description détaillée suivante, faite à titre illustratif mais non limitatif, en référence aux figures annexées parmi lesquelles :

Fig. 1 schématise un mode de réalisation d'une installation de fourniture de gaz thérapeutique selon l'invention,

Fig. 2 schématise un mode de réalisation de l'architecture interne de l'appareil de délivrance de gaz de l'installation de fourniture de gaz de Fig. 1,

Fig. 3 représente des courbes de débit et de pression obtenues pendant un cycle respiratoire du patient.

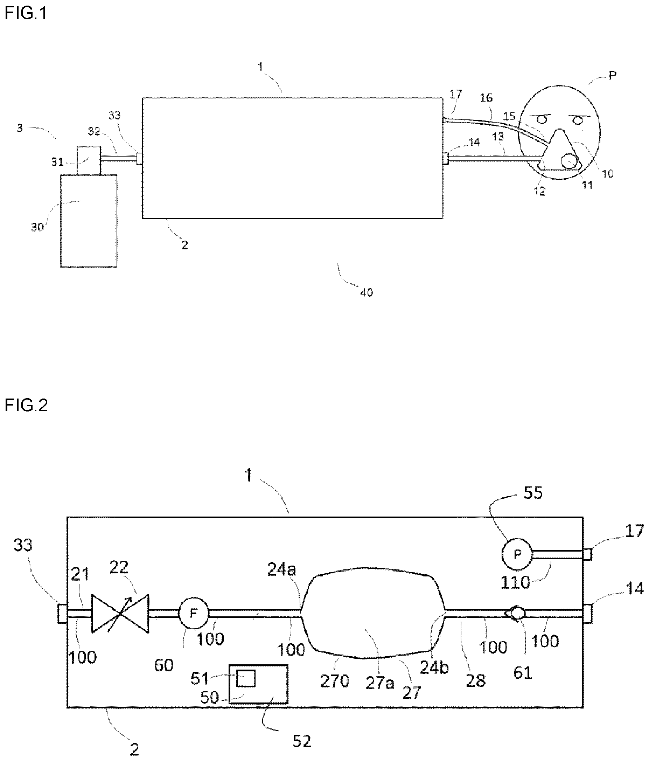

Fig. 1 est une représentation schématique d'un mode de réalisation d'une installation de fourniture de gaz thérapeutique 1 selon la présente invention.

Elle comprend un appareil de délivrance de gaz 1, détaillé en Fig. 2, comprenant un boitier externe 2 formant une carcasse rigide, par exemple en polymère, comprenant les composants internes, notamment un passage de gaz interne, un réservoir déformable, un dispositif à vanne et une unité de contrôle à microprocesseur comme expliqué ci-après.

Une source de gaz thérapeutique 3, telle qu'une bouteille de gaz 30 équipée d'une valve 31, fournit un gaz thérapeutique, c'est-à-dire un gaz ou mélange de gaz à l'appareil de délivrance de gaz 1 via un flexible de raccordement 32, connecté au port d'entrée 33 de l'appareil de délivrance de gaz 1.

Le gaz thérapeutique comprend (au moins) un composé thérapeutiquement efficace/actif, i.e. un principe actif, ayant une concentration initiale ($C_{ini}$) donnée. Par exemple, le gaz thérapeutique peut être un mélange binaire d'argon et d'oxygène ($Ar/O_2$) contenant 60% d'argon (% en volume) et le reste d'oxygène, dans lequel l'argon est le composé thérapeutiquement efficace/actif.

Le gaz thérapeutique traverse l'appareil de délivrance de gaz 1, comme expliqué ci-après, pour ensuite être délivré à un patient P par le biais d'une conduite de gaz 13 qui est raccordée fluidiquement à un port de sortie 14 du système d'administration 1. Le gaz est fourni au patient P via un masque respiratoire 10 alimenté par la conduite de gaz 13, aussi appelée tubulure, tel un tuyau flexible, laquelle conduite de gaz 13 a généralement plusieurs mètres de longueur, typiquement environ 3 ou 4 mètres, voire même plus dans certains cas.

De préférence, le masque respiratoire 10 est un masque facial, i.e. un masque naso-buccal, couvrant la bouche et le nez du patient. D'autres interfaces respiratoires analogues ou équivalentes pourraient bien entendu convenir.

Le masque 10, qui est ici facial, présente un port d'expiration 11, aussi appelé évent, communiquant fluidiquement avec l'atmosphère ambiante et un port d'inhalation 12 qui est connecté fluidiquement à la conduite de gaz 13 qui achemine le gaz. Le port d'expiration 11 comprend préférablement un clapet anti-retour qui dirige et permet l'évacuation à l'atmosphère des gaz lors de l'expiration du patient, c'est-à-dire des gaz expirés riches en $CO_2$, et empêche par ailleurs l'entrée d'air ambiant dans le masque 10 lorsque le patient inhale le gaz thérapeutique, c'est-à-dire pendant ses phases inspiratoires. Le clapet anti-retour comprend une valve unidirectionnelle, tel un disque en silicone reposant sur une surface perforée, qui ne permet au gaz de ne passer que dans un sens, par exemple la valve unidirectionnelle de référence 97351 commercialisée par la société Qosina.

Le masque 10 présente en outre un port de prise de pression 15 connecté fluidiquement à une ligne ou conduit d'amenée de pression 16, tel qu'un tuyau flexible, par exemple un tuyau en silicone de plusieurs mètres, lui-même relié pneumatiquement au capteur de pression 55, via un port de mesure 17 agencé sur le boitier 2 de l'appareil de délivrance de gaz 1. Cette configuration permet au capteur de pression 55 agencé dans le boitier 2 de réaliser des mesures de pression dans le masque 10 afin d'y suivre la pression (i.e. dépression) qui s'y exerce, comme explicité ci-après.

La source de gaz 3 contient le gaz thérapeutique sous pression, typiquement à une pression maximale de l'ordre de 250 bar, voire plus. La valve 31 est de préférence un robinet à régulateur de pression intégré ou RDI délivrant le gaz dans le flexible de raccordement 32 à une pression réduite, inférieure à 10 bar, par exemple de l'ordre de 3 à 5 bar. Le régulateur de pression intégré 31 est de préférence protégé par un chapeau rigide (non représenté).

Fig.2 schématise un mode de réalisation de l'architecture interne de l'appareil de délivrance de gaz 1 de l'installation de fourniture de gaz 40 selon la présente invention, qui est schématisée en Fig.1.

L'appareil de délivrance de gaz 1 comprend une unité de contrôle 50 comprenant un microprocesseur 51 porté par une carte électronique 52 servant notamment à piloter un dispositif à vanne 22, telle une vanne proportionnelle, pour fixer ou ajuster le débit de gaz traversant ledit dispositif à vanne 22.

L'unité de contrôle 50 comprend un (ou des) microprocesseur(s) 51, typiquement un (ou plusieurs) microcontrôleur(s), exécutant un (ou plusieurs) algorithme(s) qui reçoit et analyse les mesures fournies par différents capteurs, en particulier par le capteur de pression 55 agencé dans le boitier 2 et relié pneumatiquement au masque 10 par la ligne d'amenée de pression 16. L'unité de contrôle 50 réalise aussi des calculs, des comparaisons ou d'autres tâches comme expliqué ci-après.

Un passage de gaz interne 100, par exemple un conduit ou analogue, est arrangé dans le boitier 2 et s'étend entre un port or orifice d'entrée 33 et un port ou orifice de sortie 14 de manière à convoyer le gaz thérapeutique depuis le port d'entrée 33 vers le port de sortie 14, et permettre ensuite son acheminement jusqu'au masque 10, via le conduit flexible 13.

Le dispositif à vanne 22, à savoir ici une vanne proportionnelle, est agencé dans le passage de gaz interne 100, de préférence dans la section amont 21 dudit passage de gaz interne 100. Il est contrôlé par le microcontrôleur 51 de l'unité de contrôle 50 pour modifier le débit de gaz thérapeutique traversant ledit dispositif à vanne 22 et circulant dans le lumen du passage de gaz interne 100 en direction du port ou orifice de sortie 14, comme décrit ci-après.

Différents types de vannes proportionnelles peuvent être utilisés en tant que dispositif à vanne 22, de préférence on choisit une vanne proportionnelle fonctionnant sur une large gamme de débits, par exemple la vanne référencée IMI FAS FLATPROP.

Un capteur de débit 60 est agencé dans le passage de gaz interne 100, en sortie du dispositif à vanne 22, afin de mesurer le débit du gaz thérapeutique délivré par le dispositif à vanne 22, typiquement une vanne proportionnelle. Le capteur de débit 60 peut être un capteur de débit massique ou bien basé sur un capteur de pression différentielle.

Le capteur de débit 60 est connecté électriquement à l'unité de contrôle 50 et délivre un signal de débit qui est traité par ladite unité de contrôle 50, typiquement par le microprocesseur 51, préférentiellement un microcontrôleur.

De préférence, un débit volumétrique est obtenu après conversion du signal fourni par le capteur de débit 60 à l'aide d'une (ou des) table de correspondance spécifique mémorisée dans une mémoire coopérant avec l'unité de contrôle 50. Le capteur de débit 60 peut également être utilisé aussi pour détecter tout défaut de la vanne proportionnelle 22 ou pour déterminer la quantité de gaz, c'est-à-dire le volume, délivré par la source de gaz 3.

Le passage de gaz interne 100 achemine ensuite le gaz vers un réservoir déformable 27, en particulier un réservoir flexible, disposé en aval du capteur de débit 60, et en connexion fluidique avec ledit passage de gaz 100.

Le réservoir déformable 27 comprend une paroi périphérique flexible 270 délimitant un volume interne 27a pour le gaz, formant une poche déformable pour le gaz thérapeutique. Au repos, le volume interne 27a est compris par exemple entre 0,2 et 1 L environ.

Le débit de gaz entre dans le volume interne 27a du réservoir déformable 27 par un orifice d'entrée de réservoir 24a, en communication fluidique avec le passage de gaz intérieur 100. De préférence, les propriétés du réservoir déformable 27 sont telles qu'il est hautement déformable. Par exemple, sa paroi périphérique 270 a une épaisseur comprise entre environ 0,25 et 0,75 mm et est formée d'un silicone flexible biocompatible, par exemple du silicone de la série LSR commercialisée par NuSil.

Le gaz ressort du réservoir 27 par un orifice de sortie de réservoir 24b qui est relié fluidiquement à une section aval 28 du passage de gaz interne 100, se prolongeant jusqu'au port de sortie 14.

Un (ou plusieurs) dispositif anti-retour 61, tel qu'un clapet anti-retour, est agencé dans le passage de gaz interne 100, en aval du réservoir 27, à savoir entre l'orifice de sortie 24b du réservoir 27 et le port de sortie 14 du boitier 2, pour empêcher tout reflux de gaz. Ainsi, les gaz expirés par le patient P sont évacués uniquement par le port d'expiration 11 du masque 10 et ne peuvent pas retourner dans le réservoir 27.

Le clapet anti-retour 61 est de préférence conçu de telle sorte qu'une très faible chute de pression, typiquement inférieure ou égale à 0,2 mbar, est générée à travers lui, quand un flux de gaz le traverse.

Bien entendu, plusieurs clapets anti-retour 61 peuvent également être utilisés à la place d'un seul, par exemple 3 à 5 disposés en parallèle (non représenté).

Afin de permettre de réaliser des mesures de pression dans le masque 10, il est prévu un capteur de pression 55, de préférence un capteur de pression différentielle, dans le boitier 2 de l'appareil 1. Le capteur de pression 55 est configuré pour mesurer aussi bien des pressions négatives (c'est-à-dire des pressions inférieures à la pression atmosphérique ou dépressions) jusqu'à -5 mb environ, que positives, c'est-à-dire des pressions supérieures à la pression atmosphérique ou surpressions, jusqu'à +5 mb environ.

Le capteur de pression 55 est ici un capteur de pression différentielle qui comprend deux orifices de détection comprenant un premier orifice de détection maintenu aux conditions atmosphériques (c'est-à-dire à la pression atmosphérique, i.e. 1 atm) et un deuxième orifice de détection disposé dans un conduit de mesure 110, raccordé au port de mesure 17 du boitier 2. Le port de mesure 17 est connecté fluidiquement à la ligne d'amenée de pression 16 reliée au masque 10 pour y suivre la pression qui règne dans la chambre respiratoire dudit masque 10. Par exemple, on peut utiliser le capteur de pression différentielle référencé SDP3X disponible auprès de Sensirion.

A des intervalles de temps successifs, par exemple à une fréquence de 5 msec, le capteur de pression différentielle 55 envoie un signal de mesure de pression $P_{masque}$ à l'unité de contrôle 50, lequel signal $P_{masque}$ reflète la pression mesurée dans le masque 10 à l'instant considéré. L'unité de contrôle 50 traite alors ce signal de pression afin notamment de piloter la vanne proportionnelle 22 pour ajuster le débit gazeux envoyé au réservoir flexible 27.

Le réservoir flexible 27 présente différents états de gonflage/dégonflage en fonction de la pression de gaz qui y règne, donc de la quantité de gaz qui y est introduite ou soutirée, comprenant au moins :

- un état dit « au repos », dans lequel le volume interne 27a, rempli de gaz, est à pression atmosphérique (i.e. 1 atm).

- un état dit « gonflé », dans lequel le volume interne 27a, rempli de gaz, est à une pression supérieure à la pression ambiante (c'est-à-dire> 1 atm).
- des états de « dégonflage partiel», dans lequel une partie du gaz contenu dans le réservoir en est sorti.

Une source d'alimentation électrique (non montrée) fournit du courant électrique à tous les composants fonctionnant avec de l'énergie électrique, tels que capteurs, unité de contrôle, vannes commandée, interface homme-machine (IHM), écran d'affichage numérique.... Elle peut être disposée dans le boitier 2, par exemple une batterie rechargeable ou comprend un cordon et une prise de raccordement au secteur (110/220V), et éventuellement un convertisseur de courant.

Durant une thérapie avec administration de gaz thérapeutique, le patient P opère une succession d'inspirations et d'expirations pour inhaler le gaz thérapeutique, par exemple un mélange $O_2$/argon ou $N_2O/O_2$, et exhaler les gaz riches en $CO_2$ résultant des échanges pulmonaires.

Afin de faciliter la compréhension du fonctionnement de l'appareil 1, on considère que :

- la pression dans le volume interne 27a du réservoir 27 est égale à la pression atmosphérique, c'est-à-dire que le réservoir 27 est en position de repos.
- le réservoir 27 est rempli du gaz thérapeutique issu de la source de gaz 3.
- le patient entame une inspiration.

Lorsque le patient commence à inspirer, le port d'expiration 11 du masque 10 est fermé et une légère dépression se produit au niveau du port d'inhalation 12 du masque 10. Cette dépression se propage jusqu'au capteur de pression différentielle 55, via respectivement la ligne d'amenée de pression 16, le port de mesure 17 et le conduit de mesure 110. L'information de pression est ensuite transmise par le capteur de pression différentielle 55 à l'unité de traitement 50, en particulier au microprocesseur 51.

Par ailleurs, cette dépression se propage en parallèle dans le conduit 13, le port de sortie 14 et la section aval 28 du passage de gaz interne 100.

Alors que la pression de gaz dans le volume interne 27a du réservoir 27 est égale à la pression atmosphérique (i.e. 1 atm), une pression différentielle positive paraît alors au travers du clapet anti-retour 61 qui permet à une certaine quantité de gaz de passer à travers ledit clapet anti-retour 61 pour répondre à la demande respiratoire du patient.

Autrement dit, un débit gazeux peut s'établir depuis le réservoir 27 en direction du masque 10. Il s'ensuit que le volume interne 27a du réservoir 27 se vide alors et le réservoir 27 se dégonfle, créant à son tour une légère dépression dans le volume interne 27a.

Or, l'unité de contrôle 50 est configurée pour garantir qu'à tout moment, la pression régnant dans le masque 10 soit aussi proche que possible de la pression atmosphérique (i.e. 1 atm), soit 0 mbar relatif. Pour ce faire, l'unité de contrôle 50 pilote la vanne proportionnelle 22 de manière à ce que le débit fourni par ladite vanne proportionnelle 22 soit proportionnel à la pression mesurée $P_{masque}$ dans le masque 10 par le capteur de pression différentielle 55.

A cette fin, le microprocesseur 51 peut par exemple mettre en oeuvre un algorithme du type :

- Si « Pression masque » négative : Débit (L/min) = $\alpha * |P|$
- Si « Pression masque » positive : Débit (L/min) = 0

où : $\alpha$ est une constante positive et $|P|$ est la valeur absolue de la pression mesurée dans le masque.

L'unité de contrôle 50 n'agit donc sur la vanne proportionnelle 22 que si la pression régnant dans le masque 10 est négative, c'est-à-dire que la vanne proportionnelle 22 est commandée ou reste en position fermée dès lors que la pression dans le masque 10 devient positive.

En cas de pression négative (i.e. dépression) dans le masque 10, mesurée par le capteur de pression différentielle 55, traduisant une inspiration du patient P, la vanne proportionnelle 22 va être commandée par l'unité de contrôle 50, en particulier par le microprocesseur 51, tel que : Débit (L/min) = $\alpha * |P|$.

Il apparait alors une proportionnalité entre le débit délivré par la vanne proportionnelle 22 et la pression négative mesurée dans le masque 10 par le capteur de pression différentielle 55. Plus la valeur de pression s'écarte de 0 mb, plus le débit est élevé. A l'inverse, plus la valeur de pression se rapproche de 0 mb, plus le débit est faible. Il est bien entendu que l'algorithme décrit ici à un but illustratif, et que des algorithmes plus sophistiqués tel qu'un contrôle par termes Proportionnel - Intégral et Dérivé (PID) pourraient être implémentés.

En outre, l'appareil de délivrance de gaz 1 peut comprend d'autres éléments, comme une interface homme-machine (IHM) avec écran de visualisation d'informations, de préférence un écran tactile, une ou des touches ou boutons de sélection, un dispositif de mise en route, tel un bouton allumé/éteint, un système d'alarme et/ou d'autres éléments.

Dans une telle installation de fourniture de gaz thérapeutique 40, il a été constaté en pratique que des fuites, i.e. défauts d'étanchéité fluidique, pouvaient apparaître au niveau du masque respiratoire 10, en particulier sur le pourtour du masque 10, c'est-à-dire la zone en contact avec le visage du patient, qui peut comprendre un coussinet flexible.

Ces entrées d'air ambiant indésirables sont d'autant plus importantes que la longueur de la ligne d'amenée de gaz 13 est grande. En effet, cette ligne d'amenée de gaz 13 induit une résistance à l'inspiration augmentant avec sa longueur et obligeant alors le patient à produire une pression négative (i.e. une dépression) d'autant plus importante pour pouvoir inspirer la quantité de gaz dont il a besoin que la résistance de la ligne 13 est élevée.

Dès lors, en cas de « fuites » au niveau du masque, c'est-à-dire de défauts d'étanchéité, cette pression négative entraine de l'air ambiant qui pénètre alors dans le masque et dilue le gaz thérapeutique, pendant les phases inspiratoires du patient, ce qui est problématique car pouvant conduire à une baisse d'efficacité notable du composé actif, i.e. composé efficace, contenu dans le gaz thérapeutique, administré au patient. Ainsi, certaines propriétés thérapeutiques de l'argon disparaissent dès lors que sa concentration devient inférieure à 50 vol. %.

Pour résoudre ce problème, l'installation de l'invention 40 est configurée pour vérifier que la dilution non-désirée du gaz thérapeutique n'est pas excessive, c'est-à-dire insuffisante pour impacter négativement l'efficacité de traitement du patient et, le cas échéant, pour avertir l'utilisateur d'une dilution excessive, c'est-à-dire une concentration en composé actif insuffisante dans le gaz fourni au patient P.

Plus précisément, pour ce faire, il est prévu des moyens de mémorisation pour mémoriser une valeur-seuil de concentration ($C_{min}$) en composé thérapeutiquement actif, par exemple une mémoire flash agencée dans l'appareil.

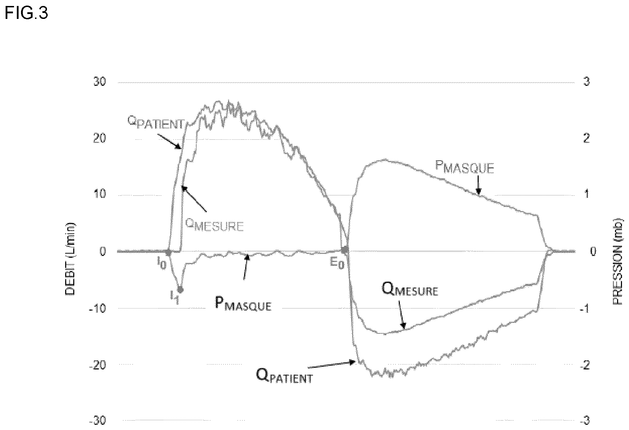

Par ailleurs, l'unité de contrôle 50 est configurée pour pouvoir estimer les fuites au niveau du masque respiratoire 10 pendant les cycles respiratoires du patient, c'est-à-dire les défauts d'étanchéité conduisant aux entrées d'air ambiant susceptibles de diluer le gaz thérapeutique et ce, afin de pouvoir avertir l'utilisateur en cas de dilution excessive du composé actif dudit gaz thérapeutique. Comme illustré en Fig. 3 et expliqué ci-après, chaque cycle respiratoire comprend une phase ou période inspiratoire (I) pendant laquelle le patient inspire/inhale du gaz qui est suivie d'une phase ou période expiratoire € pendant laquelle le patient expire/exhale du gaz enrichi en $CO_2$.

Plus précisément, l'unité de contrôle 50 détermine d'abord le débit expiré ($Q_{exp}$) s'échappant par le port expiratoire 11 du masque 10, en particulier un masque facial, à partir de la pression de gaz ($P_{masque}$) mesurée au niveau dudit masque 10, pendant une/chaque phase expiratoire du patient P, c'est-à-dire lorsque le patient expire des gaz chargés en CO2 qui sont évacués vers l'atmosphère via le port expiratoire 11 du masque 10.

Ensuite, elle détermine à partir du débit de gaz fourni ($Q_{alim}$) pendant une (chaque) phase inspiratoire du patient et du débit expiré ($Q_{exp}$) pendant la phase expiratoire suivant cette phase inspiratoire, des volumes de gaz fourni ($V_{alim}$) et expiré ($V_{exp}$), et détermine à partir de ces volumes de gaz fourni ($V_{alim}$) et expiré ($V_{exp}$), et de l'intégrale de la pression de gaz ($P_{masque}$) pendant la phase inspiratoire et de l'intégrale de la pression de gaz ($P_{masque}$) pendant la phase expiratoire, au moins un volume de fuite ($V_{fuite}$) qui entre dans le masque 10 pendant ladite phase inspiratoire. Les intégrales sont calculées par le microprocesseur de l'unité de contrôle 50.

Le volume de fuite ($V_{fuite}$) et les volumes de gaz fourni ($V_{alim}$) et expiré ($V_{exp}$), permettent alors à l'unité de contrôle 50 de calculer un taux de dilution du gaz, par exemple exprimé en pourcentage (D%).

Grâce au taux de dilution du gaz ayant été déterminé et connaissant par ailleurs la concentration initiale ($C_{ini}$) du composé thérapeutiquement actif contenu dans le gaz thérapeutique, par exemple de l'argon contenu dans un mélange $Ar/O_2$, l'unité de contrôle 50 peut calculer la concentration réelle ($C_{reel}$) en ce composé thérapeutiquement actif au sein du masque 10, c'est-à-dire la concentration réellement fournie au patient P, laquelle dépend des fuites au masque, c'est-à-dire des entrées d'air intempestives résultant des défauts d'étanchéité.

La concentration initiale ($C_{ini}$) du composé thérapeutiquement actif contenu dans le gaz thérapeutique est connue et est préférentiellement mémorisée par l'unité de contrôle 50, en particulier par le microprocesseur. Cette concentration initiale ($C_{ini}$) peut être indiquée, ajustée ou changée par l'utilisateur via une interface homme-machine ou IHM. L'IHM peut aussi servir à préciser le type de gaz thérapeutique, i.e. nature et composition d'un mélange gazeux.

En comparant la concentration réelle calculée ($C_{reel}$) à la valeur-seuil de concentration ($C_{min}$) mémorisée par les moyens de mémorisation, l'unité de contrôle 50 peut déterminer si la concentration réelle ($C_{reel}$) calculée est inférieure ou non à la valeur-seuil de concentration ($C_{min}$) et, le cas échéant, commander les moyens d'alarme pour déclencher une alarme sonore et/ou visuelle afin d'avertir l'utilisateur d'une dilution excessive résultant de fuites importantes (i.e. défauts d'étanchéité) au masque 10.

L'utilisateur peut alors vérifier le positionnement du masque 10 sur le visage du patient et éventuellement le réajuster pour minimiser ou supprimer les défauts d'étanchéité, donc les fuites.

Fig. 3 propose des courbes de débit et de pression en fonction du temps, schématisant le fonctionnement de l'unité de contrôle 50 de l'installation 40 de l'invention, en particulier de (ou des) l'algorithme mis en oeuvre par le microprocesseur 51 de l'unité de contrôle 50 de l'appareil de délivrance de gaz 1, pendant un cycle respiratoire, c'est-à-dire pendant les phases inspiratoire (I) et expiratoire (E) du patient P, afin de permettre de déterminer l'impact des fuites, c'est-à-dire donc d'évaluer/d'estimer le niveau (i.e. taux) de dilution du gaz thérapeutique par de l'air ambiant.

Ces courbes ont été obtenues sur un banc de test comprenant un « patient électronique », à savoir un dispositif mimant la respiration d'un patient, par exemple le simulateur respiratoire ASL 5000 disponible auprès de la société Ingmar Medical.

L'appareil de délivrance de gaz 1 est connecté au « patient électronique » au moyen d'un conduit d'acheminement de gaz à orifice(s) calibré(s) simulant une ou des fuites au niveau du masque respiratoire 10.

Plus précisément, Fig.3 représente un cycle respiratoire d'un patient P où :

- $Q_{PATIENT}$ est le débit de gaz respectivement inhalé et expiré par le patient au niveau de sa trachée. Ce débit ne peut être connu de l'appareil de délivrance de gaz 1.
- $P_{MASQUE}$ est la pression mesurée dans le masque respiratoire 10 par l'appareil de délivrance de gaz 1.
- $Q_{MESURE}$ est le débit mesuré par l'appareil de délivrance de gaz 1 au cours du cycle respiratoire du patient P.

L'inspiration du patient P se découpe en deux phases distinctes successives $I_0$, $I_1$ où $I_0$ correspond au tout début de l'inspiration. Ce début d'inspiration peut être détecté dès lors que la pression $P_{MASQUE}$ passe sous un seuil prédéterminé et enregistré dans l'unité de contrôle 50, par exemple une valeur strictement négative. A cet instant la pression relative dans le réservoir 27 est nulle, c'est-à-dire à la pression atmosphérique (i.e. 1 atm).

Comme susmentionné, l'inspiration du patient crée alors une dépression dans le masque 10. En réponse à cette dépression, l'unité de contrôle 50 va piloter la vanne proportionnelle 22 pour ajuster le débit de gaz thérapeutique afin de limiter la chute en pression dans le masque 10.

Or, comme dans tout système intégrant des éléments électromécaniques, il existe un temps de réponse intrinsèque, c'est-à-dire un délai, en réponse à la manifestation physique qu'est ici la dépression au masque 10.

Pendant cette phase $I_0$, la pression dans le masque 10 diminue ce qui entraine:

- une diminution de la pression dans le réservoir déformable 27, signe que ce dernier se dégonfle et qu'une quantité de gaz, i.e. un débit, circule à travers le clapet anti-retour 61 en direction du masque respiratoire 10.
- un entrainement d'air ambiant dans le masque 10, via les fuites présentes sur le pourtour du masque respiratoire 10, ce qui contribue à la dilution du gaz thérapeutique fourni par l'appareil de délivrance de gaz 1 et inhalé par le patient P.

Cette diminution de pression dans le masque respiratoire 10 atteint, en $I_1$, une valeur minimale, qui correspond au moment où l'électrovanne proportionnelle 22 commence à s'ouvrir en réponse à la sollicitation de l'unité de contrôle 50 et donc à délivrer un débit gazeux pour subvenir au besoin du patient P, accroissant ainsi la pression dans le masque respiratoire 10 et en la maintenant aussi proche que possible de 0.

Les éléments en amont du masque respiratoire 10, c'est-à-dire principalement la ligne 13 mais aussi le clapet anti-retour 61 et la section aval 28 du passage 100 (jusqu'à l'orifice de sortie 24b du réservoir 27), créent une résistance à l'écoulement du gaz.

En relation à la demande inspiratoire du patient P, c'est-à-dire son débit inspiratoire, cette résistance à l'écoulement est égale à la différence de la pression dans le réservoir 27 et de la pression dans le masque 10. Ainsi, lorsque la pression la pression dans le masque respiratoire 10 avoisine 0, alors que l'appareil de délivrance de gaz 1 fournit un débit gazeux au patient P, la pression régnant dans le réservoir 27 est positive.

Si l'appareil 1 ne disposait pas d'un mécanisme de délivrance de débit basé sur la pression régnant dans le masque 10, c'est-à-dire si le réservoir 27 se vidait progressivement en réponse à l'inspiration du patient P, alors la dépression dans le masque 10 permettant au patient P de subvenir à ses besoins ventilatoires, serait égale à la somme des résistances à l'écoulement des éléments situés en aval du réservoir 27, incluant le réservoir 27 lui-même. Ceci demanderait alors au patient de fournir un effort inspiratoire important, générant de l'inconfort, et imposerait une pression négative plus importante dans le masque respiratoire 10, accentuant alors l'apport d'air ambiant par les fuites autour dudit masque respiratoire 10, et diminuant ainsi la concentration de gaz thérapeutique inhalée par le patient.

L'inspiration du patient P laisse ensuite place à une phase expiratoire $E_0$ où le patient expire à travers le port expiratoire 11 du masque 10. Cette expiration génère alors une pression positive dans le masque 10 et l'unité de contrôle 50 commande alors la vanne proportionnelle 22 de manière à interrompre la délivrance de gaz, c'est-à-dire le débit.

De façon analogue à la détection de l'inspiration $I_0$, on peut considérer que la détermination du passage à l'expiration se fait lorsque la pression $P_{MASQUE}$ passe au-dessus d'un seuil prédéterminé et enregistré dans l'unité de contrôle 50, par exemple une valeur strictement positive.

Le port expiratoire 11 du masque 10 présente une résistance à l'écoulement, i.e. à un débit. Sachant que ce port expiratoire 11 débouche à l'air ambiant, c'est-à-dire à la pression atmosphérique, il existe une relation entre la pression $P_{MASQUE}$ mesurée dans le masque respiratoire 10 et le débit circulant dans ledit port expiratoire 11. Dès lors, en ayant au préalable enregistré une table de conversion dans l'unité de contrôle 50, le microprocesseur 51 est en mesure de déterminer le débit $Q_{MESURE}$ s'échappant par le port expiratoire 11, via l'analyse en temps réel de la pression $P_{MASQUE}$ régnant dans le masque respiratoire 10.

La séquence suivante vise à démontrer la capacité de l'appareil de délivrance de gaz respiratoire 1 à déterminer la concentration de gaz thérapeutique inhalée par le patient P et de s'assurer que celle-ci est supérieure à une valeur minimale donnée préenregistrée, i.e. mémorisée, à savoir une concentration minimale en composé actif dans le gaz thérapeutique et ce, malgré la dilution causée par les entrées d'air dues aux défauts d'étanchéité susmentionnés.

Autrement dit, l'appareil de délivrance de gaz respiratoire 1 de l'installation 40 de l'invention permet de s'assurer que la dilution n'est pas excessive de la manière suivante.

Pendant la phase $I_0$-$E_0$ correspondant à l'inspiration du patient P, on peut considérer l'équation suivante :

$$Q_{PATIENT} = Q_{MESURE} + Q_{FUITE}$$

où :

- $Q_{PATIENT}$ est le débit inhalé par le patient,
- $Q_{MESURE}$ est le débit délivré par l'électrovanne proportionnelle 22 et mesuré par le capteur de débit 60,
- $Q_{FUITE}$ est le débit introduit par les fuites (dues aux défauts d'étanchéité) sur le pourtour du masque respiratoire 10 lors de cette phase inspiratoire.

Or, le débit de fuite $Q_{FUITE}$ peut également être vue comme un élément permettant à un gaz de circuler, et dès lors présentant une résistance à l'écoulement. Du fait que la pression ambiante règne à l'extérieur du masque respiratoire 10, il peut être considéré que le débit introduit par les fuites $Q_{FUITE}$ est tel que :

$$Q_{FUITE} = -\beta . P_{MASQUE}$$

où :

- $\beta$ est une constante, inconnue,
- $P_{MASQUE}$ est la pression régnant dans le masque respiratoire 10. Quand $P_{MASQUE}$ est négative, $Q_{FUITE}$ est positif.

De là, on obtient :

$$Q_{PATIENT} = Q_{MESURE} - \beta . P_{MASQUE}$$

En calculant l'intégrale de ces grandeurs, pendant l'inspiration du patient P, c'est-à-dire sur l'intervalle $[I_0, E_0]$, on obtient alors

$$V_{PATIENT\ I} = V_{MESURE\ I} - \beta \int_{I0}^{E0} P_{MASQUE}$$

où :

- $V_{PATIENT\ I}$ est le volume inhalé par le patient pendant la phase inspiratoire,
- $V_{MESURE\ I}$ est le volume délivré par l'appareil de délivrance de gaz 1, calculé par le microprocesseur 51 en réponse aux données fournies par le capteur de débit 60.

De façon analogue, l'expiration du patient P sur l'intervalle $E_0$-$I_0$ (considérant $I_0$ la détection de l'inspiration successive à cette expiration), on a:

$$Q_{PATIENT} = Q_{MESURE} + Q_{FUITE}$$

où :

- $Q_{PATIENT}$ est le débit expiré par le patient,
- $Q_{MESURE}$ est le débit mesuré par le microprocesseur 51 selon la courbe enregistré dans l'unité de contrôle 50, mettant en relation la pression $P_{MASQUE}$ régnant dans le masque respiratoire 10 et le débit traversant le port expiratoire 11.

- $Q_{FUITE}$ est le débit s'échappant des fuites du masque respiratoire 10 lors de la phase expiratoire, du fait de la positivité de la pression $P_{MASQUE}$.

En considérant que la constante β reste identique entre les phases inspiratoire et expiratoire, c'est-à-dire que le positionnement du masque 10 ne change pas, on obtient, par un raisonnement similaire que :

$$V_{PATIENT\ E} = V_{MESURE\ E} + \beta \int_{E0}^{I0} P_{MASQUE}$$

Or, de façon générale, le volume inspiré par un patient P est équivalent au volume expiré, d'où :

$$V_{MESURE\ I} - \beta \int_{I0}^{E0} P_{MASQUE} = V_{MESURE\ E} + \beta \int_{E0}^{I0} P_{MASQUE}$$

Et donc β devient calculable sous la forme:

$$\beta = \frac{V_{MESURE\ I} - V_{MESURE\ E}}{\int_{E0}^{I0} P_{MASQUE} + \int_{I0}^{E0} P_{MASQUE}}$$

Dès lors, si : $V_{PATIENT\ I} = V_{MESURE\ I} - \beta \int_{I0}^{E0} P_{MASQUE}$ il est alors possible de déterminer le taux (D%) de dilution, en termes de volumes de ce que le patient a inhalé par rapport à ce que l'appareil de délivrance de gaz 1 a fourni, soit le taux de dilution donné par l'équation suivante :

$$D\% = \frac{V_{MESURE\ I}}{V_{MESURE\ I} - \beta \int_{I0}^{E0} P_{MASQUE}}$$

D% est exprimé ici en pourcentage.

Par exemple, le microprocesseur 51 est en capacité de déterminer que le volume de gaz $V_{MESURE\ I}$ fourni par l'appareil de délivrance 1 pendant l'inspiration est de l'ordre de 440mL, alors que le volume expiré $V_{MESURE\ E}$, c'est-à-dire le volume de gaz s'échappant par le port expiratoire 11 du masque, est de l'ordre de 300mL, ce qui pourrait signifier que la fuite sur le pourtour du masque 10 est relativement importante puisque la différence entre ces deux volumes est de l'ordre de 140mL.

Par ailleurs, la pression dans le masque respiratoire 10 pendant la phase inspiratoire est négative, c'est-à-dire qu'un volume supplémentaire va être introduit par cette fuite pendant ladite phase inspiratoire. De la même manière, du fait de la pression positive régnant dans le masque respiratoire 10 pendant la phase expiratoire, une partie du volume expiré par le patient P va s'échapper à l'ambient par cette même fuite.

Or, étant donné que la pression dans le masque respiratoire 10 reste très proche de 0 lors de la phase inspiratoire, l'apport du volume de fuite pendant la phase inspiratoire est relativement faible, au regard du volume s'échappant par cette même fuite pendant la phase expiratoire, car la pression régnant dans le masque respiratoire 10 y est bien plus prononcée, i.e. bien plus élevée. En analysant l'évolution des débit et pression pendant le cycle respiratoire (i.e. l'inspiration et l'expiration) et conformément à l'équation développée ci-dessus, le microprocesseur 51 peut déterminer que le volume introduit par cette fuite pendant la phase inspiratoire se limite à seulement 20mL, et donc de calculer le pourcentage de dilution D% (calculé en volume), soit environ 96.3% de la concentration initiale ($C_{ini}$) en composé actif du gaz thérapeutique.

Ainsi, si l'on prend l'exemple d'un mélange $Ar/O_2$ contenant une concentration initiale ($C_{ini}$) en argon égale à env. 60 vol.% provenant de la source de gaz 3, la concentration réelle en argon (composé actif) du gaz thérapeutique déterminée par le microprocesseur 51 est alors de l'ordre de 57 vol. %, donc au-dessus de la valeur-seuil de 50% en dessous de laquelle l'argon n'est plus efficace dans certains traitements, ce qui garantit l'efficacité du mélange $Ar/O_2$ malgré sa dilution par de l'air ambiant provenant des fuites.

D'une façon générale, en opérant en (quasi)continu de tels mesures et calculs, c'est-à-dire de préférence pour chaque cycle respiratoire du patient, l'appareil de délivrance de gaz 1, notamment l'unité de contrôle 50 à microprocesseur 51, est en mesure de déterminer si la concentration réelle ($C_{reel}$) en composé actif dans le gaz thérapeutique est ou non au-dessus d'une valeur-seuil de concentration ($C_{min}$) en composé thérapeutiquement actif, laquelle est mémorisée par les moyens de mémorisation, par exemple une mémoire flash ou directement dans le microprocesseur 51.

Lorsque la concentration réelle ($C_{reel}$) devient inférieure à la valeur-seuil de concentration ($C_{min}$) en composé thérapeutiquement actif mémorisée, par exemple inférieure à 50 vol.% dans l'exemple de l'argon, le microprocesseur 51 est configuré pour commander des moyens d'alarme et ainsi déclencher une alarme sonore et/ou visuelle destinée à avertir l'utilisateur qu'une dilution excessive se produit, ce qui équivaut à des fuites importantes signifiant souvent que le masque 10 du patient P est mal positionné

Préférentiellement, l'unité de contrôle 50 et microprocesseur 51 peuvent limiter le déclenchement de fausses alarmes en calculant une ou des moyennes à partir de valeurs de concentrations obtenues sur plusieurs cycles et en travaillant sur ces moyennes plutôt que sur des valeurs instantanées.

Les moyens d'alarme permettant de déclencher l'alarme sonore et/ou visuelle comprennent par exemple un haut-parleur pour diffuser un signal audio et/ou une (des) diode lumineuse, par exemple de type LED, pour fournir un signal visuel, par exemple un clignotement de la LED, ou autres.

D'une façon générale, l'installation de l'invention peut être utilisée pour opérer une administration à un patient, un gaz thérapeutique formé d'un mélange de plusieurs constituants gazeux, par exemple un mélange d'argon et d'oxygène (60 vol.% Ar/40 vol.% $O_2$), devant être inhalé avant et pendant, voire après, une procédure de thrombectomie mécanique visant à traiter un accident vasculaire cérébral ou AVC chez l'être humain.

## Revendications

**1.** Installation de fourniture de gaz thérapeutique (40) comprenant :

- une source de gaz (3) comprenant au moins un composé thérapeutiquement actif à une concentration initiale ($C_{ini}$) donnée,
- un appareil de délivrance de gaz (1) alimenté en gaz par ladite source de gaz (3), et
- une interface respiratoire (10) munie d'au moins un port expiratoire (11) alimenté en gaz par ledit appareil de délivrance de gaz (1),

et dans laquelle l'appareil de délivrance de gaz (1) comprend :

- un passage de gaz interne (100) en communication fluidique avec un réservoir déformable (27) pour alimenter le réservoir déformable (27) en gaz,
- un dispositif à vanne (22) agencée sur le passage de gaz interne (100), en amont du réservoir déformable (27), pour contrôler le débit de gaz circulant dans le passage de gaz interne (100),
- une unité de contrôle (50) à microprocesseur (51) pilotant le dispositif à vanne (22) pour contrôler le débit de gaz traversant le dispositif à vanne (22) et alimentant le réservoir déformable (27) en gaz,
- un capteur de pression (55) configuré pour opérer une ou des mesures de pression de gaz ($P_{masque}$) au niveau de l'interface respiratoire (10) et fournir à l'unité de contrôle (50) la ou lesdites mesures de pression de gaz ($P_{masque}$),
- un capteur de débit (60) agencé dans le passage de gaz interne (100) pour mesurer le débit de gaz fourni ($Q_{alim}$) circulant dans ledit passage de gaz interne (100) et fournir la ou lesdites mesures de débit de gaz (Q) à l'unité de contrôle (50), et
- des moyens d'alarme,

**caractérisée en ce que** :

- l'appareil de délivrance de gaz (1) comprend en outre des moyens de mémorisation pour mémoriser une valeur-seuil de concentration ($C_{min}$) en composé thérapeutiquement actif et
- l'unité de contrôle (50) est configurée pour :

i) déterminer le débit expiré ($Q_{exp}$) s'échappant par le port expiratoire (11) de l'interface respiratoire (10) à partir de la pression de gaz ($P_{masque}$) mesurée au niveau de l'interface respiratoire (10), pendant au moins une phase expiratoire dudit patient,
ii) déterminer à partir du débit de gaz fourni ($Q_{alim}$) pendant une phase inspiratoire du patient et du débit

expiré ($Q_{exp}$) pendant la phase expiratoire suivant ladite phase inspiratoire du patient, des volumes de gaz fourni ($V_{alim}$) et expiré ($V_{exp}$),

iii) déterminer à partir des volumes de gaz fourni ($V_{alim}$) et expiré ($V_{exp}$), de l'intégrale de la pression de gaz ($P_{masque}$) pendant la phase inspiratoire et de l'intégrale de la pression de gaz ($P_{masque}$) pendant la phase expiratoire, au moins un volume de fuite ($V_{fuite}$) entrant dans l'interface respiratoire (10) pendant ladite phase inspiratoire,

iv) déterminer à partir du volume de fuite ($V_{fuite}$) et des volumes de gaz fourni ($V_{alim}$) et expiré ($V_{exp}$), un taux de dilution du gaz,

v) calculer la concentration réelle ($C_{reel}$) en ledit au moins un composé thérapeutiquement actif dans de l'interface respiratoire (10) à partir de la concentration initiale ($C_{ini}$) dudit au moins un composé thérapeutiquement actif et du taux de dilution du gaz déterminé,

vi) comparer la concentration réelle calculée ($C_{reel}$) à la valeur-seuil de concentration ($C_{min}$) en composé thérapeutiquement actif mémorisée par les moyens de mémorisation, et

vii) commander les moyens d'alarme pour déclencher une alarme sonore et/ou visuelle lorsque la concentration réelle ($C_{reel}$) calculée est inférieure à ladite valeur-seuil de concentration ($C_{min}$) en composé thérapeutiquement actif mémorisée

2. Installation selon la revendication 1, **caractérisée en ce que** l'interface respiratoire (10) est un masque respiratoire, de préférence un masque facial.

3. Installation selon la revendication 1, **caractérisée en ce que** l'unité de contrôle (50) de l'appareil de délivrance de gaz (1) est configurée pour déterminer le débit expiré ($Q_{exp}$) à partir de la pression de gaz ($P_{masque}$) mesurée au niveau de l'interface respiratoire (10) et d'au moins une table de conversion mémorisée.

4. Installation selon la revendication 1, **caractérisée en ce que** la source de gaz (3) comprend de l'argon en tant que composé thérapeutiquement actif à une concentration initiale ($C_{ini}$) supérieure à 50% en volume, de préférence un mélange argon/oxygène.

5. Installation selon la revendication 4, **caractérisée en ce que** la valeur-seuil de concentration ($C_{min}$) mémorisée est égale à 50% en volume.

6. Installation selon la revendication 1, **caractérisée en ce que** le capteur de pression (55) comprend un capteur de pression différentiel.

7. Installation selon les revendications 1 ou 6, **caractérisée en ce que** le capteur de pression (55) est configuré ou contrôlé pour opérer des mesures de pression ($P_{masque}$) à des intervalles de temps donnés, de préférence toutes les 20 msec ou moins.

8. Installation selon la revendication 1, **caractérisée en ce que** le dispositif à vanne (22) comprend une vanne proportionnelle.

9. Installation selon les revendications 1 ou 8, **caractérisée en ce que** le capteur de débit (60) est agencé dans le passage de gaz interne, en aval du dispositif à vanne (22) et/ou en amont du réservoir déformable (27).

10. Installation selon les revendications 1 ou 9, **caractérisée en ce que** le capteur de débit (60) comprend un capteur de débit massique ou un capteur de pression différentielle.

11. Installation selon la revendication 1, **caractérisée en ce que** l'unité de contrôle (50) est configurée pour commander le dispositif à vanne (22) pour augmenter le débit de gaz thérapeutique traversant ledit dispositif à vanne (22) et alimentant le réservoir déformable (27), lorsque l'unité de contrôle (50) détermine que la pression de gaz ($P_{masque}$) mesurée dans l'interface respiratoire (10) est inférieure ou égale ($P_{masque} \leq P_{seuil}$) à une valeur-seuil de pression donnée ($P_{seuil}$).

12. Installation selon la revendication 11, **caractérisée en ce que** la valeur-seuil de pression ($P_{seuil}$) est inférieure ou égale à 0 mbar.

13. Installation selon l'une des revendications 11 ou 12, **caractérisée en ce que** la valeur-seuil de pression ($P_{seuil}$) est inférieure ou égale à -0.25 mbar, de préférence inférieure ou égale à -0.5 mbar.

**14.** Installation selon l'une des revendications 11 à 13, **caractérisée en ce que** la valeur-seuil de pression ($P_{seuil}$) est mémorisée au sein de l'unité de contrôle (50).

**15.** Installation selon l'une des revendications 11 à 14, **caractérisée en ce que** la valeur-seuil de pression ($P_{seuil}$) est réglable.

**Patentansprüche**

**1.** Anlage zur Bereitstellung von therapeutischem Gas (40) umfassend:

- eine Gasquelle (3), die wenigstens einen therapeutisch aktiven Bestandteil in einer bestimmten Ausgangs-konzentration ($C_{ini}$) umfasst,
- eine Gasabgabevorrichtung (1), die von der Gasquelle (3) mit Gas versorgt wird, und
- eine Atemschnittstelle (10), die mit wenigstens einem Expirationsport (11) versehen ist, der von der Gasabgabevorrichtung (1) mit Gas versorgt wird,

und wobei die Gasabgabevorrichtung (1) Folgendes umfasst:

- einen inneren Gasdurchgang (100), der mit einem verformbaren Vorratsbehälter (27) in Fluidverbindung steht, um den verformbaren Vorratsbehälter (27) mit Gas zu versorgen,
- eine Ventilvorrichtung (22), die am inneren Gasdurchgang (100) stromauf des verformbaren Vorratsbehälters (27) angeordnet ist, um den Durchsatz von Gas zu steuern, das im inneren Gasdurchgang (100) zirkuliert,
- eine Steuereinheit (50) mit Mikroprozessor (51), die die Ventilvorrichtung (22) ansteuert, um den Durchsatz von Gas zu steuern, das die Ventilvorrichtung (22) durchströmt und den verformbaren Vorratsbehälter (27) mit Gas versorgt,
- einen Drucksensor (55), der dazu ausgebildet ist, eine oder mehrere Gasdruckmessungen ($P_{Maske}$) an der Atemschnittstelle (10) vorzunehmen und die Gasdruckmessung(en) ($P_{Maske}$) an die Steuereinheit (50) zu liefern,
- einen Durchsatzsensor (60), der im inneren Gasdurchgang (100) angeordnet ist, um den Durchsatz von bereitgestelltem Gas ($Q_{alim}$) zu messen, das im inneren Gasdurchgang (100) zirkuliert, und die Gasdurchsatzmessung(en) (Q) an die Steuereinheit (50) zu liefern, und
- Alarmmittel,

**dadurch gekennzeichnet, dass**:

- die Gasabgabevorrichtung (1) ferner Speichermittel umfasst, um einen Schwellenwert der Konzentration ($C_{min}$) des therapeutisch aktiven Bestandteils zu speichern und
- die Steuereinheit (50) dazu ausgebildet ist:

i) den Ausatmungsdurchsatz ($Q_{exp}$), der durch den Expirationsport (11) der Atemschnittstelle (10) ausströmt, anhand des Gasdrucks ($P_{Maske}$), der an der Atemschnittstelle (10) gemessen wird, während wenigstens einer Ausatmungsphase des Patienten zu bestimmen,
ii) anhand des Durchsatzes von bereitgestelltem Gas ($Q_{alim}$) während einer Einatmungsphase des Patienten und des Ausatmungsdurchsatzes ($Q_{exp}$) während der Ausatmungsphase, die auf die Einatmungsphase des Patienten folgt, Volumen von bereitgestelltem Gas ($V_{alim}$) und ausgeatmetem Gas ($V_{exp}$) zu bestimmen,
iii) anhand der Volumen von bereitgestelltem ($V_{alim}$) und ausgeatmetem Gas ($V_{exp}$), des Integrals des Gasdrucks ($P_{Maske}$) während der Einatmungsphase und des Integrals des Gasdrucks ($P_{Maske}$) während der Ausatmungsphase, wenigstens ein Leckagevolumen ($V_{leckage}$) zu bestimmen, das während der Einatmungsphase in die Atemschnittstelle (10) eintritt,
iv) anhand des Leckagevolumens ($V_{leckage}$) und der Volumen von bereitgestelltem Gas ($V_{alim}$) und ausge-atmetem Gas ($V_{exp}$) einen Verdünnungsgrad des Gases zu bestimmen,
v) die Ist-Konzentration ($C_{ist}$) des wenigstens einen therapeutisch aktiven Bestandteils in der Atemschnittstelle (10) anhand der Ausgangskonzentration ($C_{ini}$) des wenigstens einen therapeutisch aktiven Bestandteils und des bestimmten Verdünnungsgrads des Gases zu berechnen,
vi) die berechnete Ist-Konzentration ($C_{ist}$) mit dem Schwellenwert der Konzentration ($C_{min}$) des therapeu-tisch aktiven Bestandteils zu vergleichen, der von den Speichermitteln gespeichert ist, und
vii) die Alarmmittel zu steuern, um einen akustischen und/oder visuellen Alarm auszulösen, wenn die be-rechnete Ist-Konzentration ($C_{ist}$) niedriger als der gespeicherte Schwellenwert der Konzentration ($C_{min}$)

des therapeutisch aktiven Bestandteils ist

**2.** Anlage nach Anspruch 1, **dadurch gekennzeichnet, dass** die Atemschnittstelle (10) eine Atemmaske, vorzugsweise eine Gesichtsmaske ist.

**3.** Anlage nach Anspruch 1, **dadurch gekennzeichnet, dass** die Steuereinheit (50) der Gasabgabevorrichtung (1) dazu ausgebildet ist, den Ausatmungsdurchsatz ($Q_{exp}$) anhand des Gasdrucks ($P_{Maske}$), der an der Atemschnittstelle (10) gemessen wird, und wenigstens einer gespeicherten Umwandlungstabelle zu bestimmen.

**4.** Anlage nach Anspruch 1, **dadurch gekennzeichnet, dass** die Gasquelle (3) Argon als therapeutisch aktiven Bestandteil in einer Ausgangskonzentration ($C_{ini}$) größer als 50 Volumen-%, vorzugsweise ein Argon/SauerstoffGemisch umfasst.

**5.** Anlage nach Anspruch 4, **dadurch gekennzeichnet, dass** der gespeicherte Konzentrations-Schwellenwert ($C_{min}$) gleich 50 Volumen-% ist.

**6.** Anlage nach Anspruch 1, **dadurch gekennzeichnet, dass** der Drucksensor (55) einen Differenzdrucksensor umfasst.

**7.** Anlage nach den Ansprüchen 1 oder 6, **dadurch gekennzeichnet, dass** der Drucksensor (55) so ausgebildet ist oder gesteuert wird, dass er Druckmessungen ($P_{Maske}$) in bestimmten Zeitintervallen vornimmt, vorzugsweise alle 20 msec oder weniger.

**8.** Anlage nach Anspruch 1, **dadurch gekennzeichnet, dass** die Ventilvorrichtung (22) ein Proportionalventil umfasst.

**9.** Anlage nach den Ansprüchen 1 oder 8, **dadurch gekennzeichnet, dass** der Durchsatzsensor (60) im inneren Gasdurchgang stromab der Ventilvorrichtung (22) und/oder stromauf des verformbaren Vorratsbehälters (27) angeordnet ist.

**10.** Anlage nach den Ansprüchen 1 oder 9, **dadurch gekennzeichnet, dass** der Durchsatzsensor (60) einen Massendurchsatzsensor oder einen Differenzdrucksensor umfasst.

**11.** Anlage nach Anspruch 1, **dadurch gekennzeichnet, dass** die Steuereinheit (50) dazu ausgebildet ist, die Ventilvorrichtung (22) zu steuern, um den Durchsatz von therapeutischem Gas, das die Ventilvorrichtung (22) durchströmt und den verformbaren Vorratsbehälter (27) versorgt, zu erhöhen, wenn die Steuereinheit (50) bestimmt, dass der Gasdruck ($P_{Maske}$), der in der Atemschnittstelle (10) gemessen wird, kleiner als oder gleich ($P_{Maske} \leq P_{Schwelle}$) einem bestimmten Druckschwellenwert ($P_{Schwelle}$) ist.

**12.** Anlage nach Anspruch 11, **dadurch gekennzeichnet, dass** der Druckschwellenwert ($P_{Schwelle}$) kleiner als oder gleich 0 mbar ist.

**13.** Anlage nach einem der Ansprüche 11 oder 12, **dadurch gekennzeichnet, dass** der Druckschwellenwert ($P_{Schwelle}$) kleiner als oder gleich -0.25 mbar, vorzugsweise kleiner als oder gleich -0.5 mbar ist.

**14.** Anlage nach einem der Ansprüche 11 bis 13, **dadurch gekennzeichnet, dass** der Druckschwellenwert ($P_{Schwelle}$) innerhalb der Steuereinheit (50) gespeichert ist.

**15.** Anlage nach einem der Ansprüche 11 bis 14, **dadurch gekennzeichnet, dass** der Druckschwellenwert ($P_{Schwelle}$) einstellbar ist.

**Claims**

**1.** Installation (40) for supplying therapeutic gas, comprising:

- a source (3) of gas comprising at least one therapeutically active compound at a given initial concentration ($C_{ini}$),
- a gas delivery apparatus (1) fed with gas by said gas source (3), and
- a respiratory interface (10) provided with at least one exhalation port (11) which is fed with gas by said gas delivery apparatus (1),

and in which the gas delivery apparatus (1) comprises:

- an internal gas passage (100) in fluidic communication with a deformable reservoir (27) in order to feed the deformable reservoir (27) with gas,
- a valve device (22) arranged on the internal gas passage (100), upstream of the deformable reservoir (27), in order to control the flow rate of gas circulating in the internal gas passage (100),
- a control unit (50) with microprocessor (51) which controls the valve device (22) in order to control the flow rate of gas passing through the valve device (22) and feeding the deformable reservoir (27) with gas,
- a pressure sensor (55) configured to perform one or more gas pressure measurements ($P_{mask}$) at the respiratory interface (10) and to supply said gas pressure measurement (s) ($P_{mask}$) to the control unit (50) ,
- a flow rate sensor (60) arranged in the internal gas passage (100) in order to measure the flow rate of supplied gas ($Q_{alim}$) circulating in said internal gas passage (100) and to supply said gas flow rate measurement(s) (Q) to the control unit (50), and
- alarm means,

**characterized in that**:

- the gas delivery apparatus (1) additionally comprises storage means for storing a concentration threshold value ($C_{min}$) of therapeutically active compound, and
- the control unit (50) is configured to:

i) determine the exhaled flow rate ($Q_{exp}$) escaping through the exhalation port (11) of the respiratory interface (10) on the basis of the gas pressure ($P_{mask}$) measured at the respiratory interface (10), during at least one expiratory phase of said patient,

ii) determine, on the basis of the flow rate of supplied gas ($Q_{alim}$) during an inspiratory phase of the patient and of the exhaled flow rate ($Q_{exp}$) during the expiratory phase following said inspiratory phase of the patient, volumes of gas supplied ($V_{alim}$) and exhaled ($V_{exp}$) ,

iii) determine, on the basis of the volumes of gas supplied ($V_{alim}$) and exhaled ($V_{exp}$) , from the integral of the gas pressure ($P_{mask}$) during the inspiratory phase and the integral of the gas pressure ($P_{mask}$) during the expiratory phase, at least one leakage volume ($V_{leak}$) entering the respiratory interface (10) during said inspiratory phase,

iv) determine, on the basis of the leakage volume ($V_{leak}$) and the volumes of gas supplied ($V_{alim}$) and exhaled ($V_{exp}$), a rate of dilution of the gas,

v) calculate the actual concentration ($C_{actual}$) of said at least one therapeutically active compound in the respiratory interface (10) on the basis of the initial concentration ($C_{ini}$) of said at least one therapeutically active compound and of the determined rate of dilution of the gas,

vi) compare the calculated actual concentration ($C_{actual}$) to the concentration threshold value ($C_{min}$) of therapeutically active compound, stored by the storage means, and

vii) order the alarm means to trigger an acoustic and/or visual alarm when the calculated actual concentration ($C_{actual}$) is below said stored concentration threshold value ($C_{min}$) of therapeutically active compound.

2. Installation according to Claim 1, **characterized in that** the respiratory interface (10) is a breathing mask, preferably a face mask.

3. Installation according to Claim 1, **characterized in that** the control unit (50) of the gas delivery apparatus (1) is configured to determine the exhaled flow rate ($Q_{exp}$) on the basis of the gas pressure ($P_{mask}$) measured at the respiratory interface (10) and of at least one stored conversion table.

4. Installation according to Claim 1, **characterized in that** the gas source (3) comprises argon as therapeutically active compound at an initial concentration ($C_{ini}$) of greater than 50% by volume, preferably an argon/oxygen mixture.

5. Installation according to Claim 4, **characterized in that** the concentration threshold value ($C_{min}$) stored is equal to 50% by volume.

6. Installation according to Claim 1, **characterized in that** the pressure sensor (55) comprises a differential pressure sensor.

7. Installation according to Claim 1 or 6, **characterized in that** the pressure sensor (55) is configured or controlled to perform pressure measurements ($P_{mask}$) at given time intervals, preferably every 20 msec or less.

8. Installation according to Claim 1, **characterized in that** the valve device (22) comprises a proportional valve.

9. Installation according to Claim 1 or 8, **characterized in that** the flow rate sensor (60) is arranged in the internal gas passage, downstream of the valve device (22) and/or upstream of the deformable reservoir (27).

10. Installation according to Claim 1 or 9, **characterized in that** the flow rate sensor (60) comprises a mass flow rate sensor or a differential pressure sensor.

11. Installation according to Claim 1, **characterized in that** the control unit (50) is configured to control the valve device (22) to increase the flow rate of therapeutic gas passing through said valve device (22) and feeding the deformable reservoir (27), when the control unit (50) determines that the gas pressure ($P_{mask}$) measured in the respiratory interface (10) is less than or equal to ($P_{mask} \leq P_{threshold}$) at a given pressure threshold value ($P_{threshola}$).

12. Installation according to Claim 11, **characterized in that** the pressure threshold value ($P_{threshold}$) is less than or equal to 0 mbar.

13. Installation according to either of Claims 11 and 12, **characterized in that** the pressure threshold value ($P_{threshold}$) is less than or equal to -0.25 mbar, preferably less than or equal to -0.5 mbar.

14. Installation according to one of Claims 11 to 13, **characterized in that** the pressure threshold value ($P_{threshold}$) is stored in the control unit (50) .

15. Installation according to one of Claims 11 to 14, **characterized in that** the pressure threshold value ($P_{threshold}$) is adjustable.

EP 4 026 577 B1

FIG.1

FIG.2

20

FIG.3

RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 3701992 A